# EUROPEAN PATENT SPECIFICATION

(11) **EP 1 414 822 B1**
(45) Date of publication and mention of the grant of the patent: **16.02.2005**
(21) Application number: 02760197.0
(22) Date of filing: 26.06.2002
(51) Int. Cl.: C07D 473/00

(54) **IMPROVED SYNTHESIS OF BRANCHED ACYCLIC NUCLEOSIDES**
VERBESSERTE SYNTHESE VERZWEIGTER ACYCLISCHER NUKLEOSIDE
SYNTHESE AMELIOREE DE NUCLEOSIDES ACYCLIQUES RAMIFIES

(30) Priority: 26.06.2001 SE 0102273
(43) Date of publication of application: 06.05.2004
(73) Proprietor: MEDIVIR AB, 14940 Huddinge (SE)
(72) Inventor: JANSEN, Gert, DK-3520 Farum (DK)
(74) Representative: Dehmel, Albrecht, Dr.
(86) International application number: PCT/EP2002/007084
(87) International publication number: WO 2003/002564

(56) References cited:
- WO-A-00/08025
- WO-A-98/34917

## Description

### Technical field

This invention relates to improvements in the synthesis of branched acyclic nucleosides such as valomaciclovir stearate. In particular the invention provides for an improved acetal hydrolysis and concomitant reduction of the aldehyde to an alcohol in respect of key intermediates in the synthesis of such branched acyclic nucleosides.

### Background Art

Valomaciclovir stearate of the formula XX below is an acyclic nucleoside analogue, useful in the treatment of VZV and other herpesviruses and HIV.

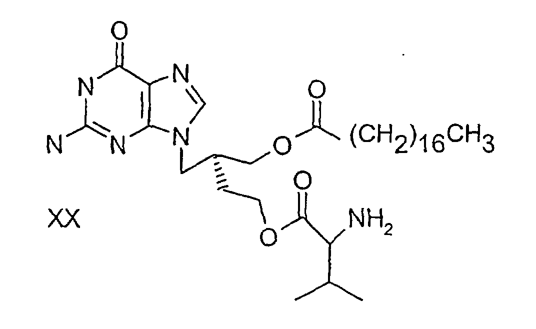

Our earlier patent applications WO98/34917, US 6 184 376 and WO00/08025 describe various synthesis routes, of which several go through a key acetal intermediate which can be generalised to the following formula I/II: 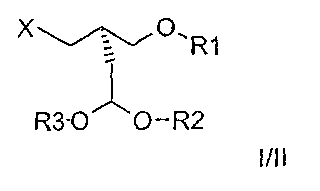 where X is a leaving group or an optionally protected guanine moiety,
R1 is hydrogen, an hydroxy protecting group or a -C(=O)C1-C22 alkyl ester
R2 and R3 are independently lower alkyl or benzyl, or R2 and R3 taken together are -CH₂CH₂- or-CH2CH2CH2- or-CH2CH2CH2CH2-.

In the prior art processes, (see for example page 30 lines 1-24 of WO98/34917) these acetals are hydrolysed by the addition of an acid such as triflic acid, HCl, acetic acid or sulphuric acid or an acid resin such as Amberlyst 15. The thus formed aldehyde is then reduced to the corresponding alcohol by the addition of an aldehyde reducing agent such as sodium borohydride, RaNi/H₂ or borane t-butylamine complex). However these reducing agents are well known to require a non-acidic operating environment, as specifically brought out at page 30 line 8 and Examples 14 & 30 of WO98/34917 and Example 2 of W000/08025. Accordingly the prior art processes require the addition of a base such as sodium bicarbonate or potassium carbonate or triethylamine or pyridine or KOH and the like to neutralise the acid which has been used for acetal hydrolysis, prior to addition of the aldehyde reducing agent.

### Brief description

We have now discovered that, even without purifying the aldehyde, dramatically improved purity of the resultant alcohol can be obtained if a borane or borohydride aldehyde reducing agent is introduced under acid conditions.

Accordingly, the invention provides a method comprising the acid hydrolysis of an intermediate of the formula II: 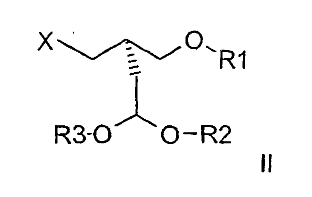 where X is a leaving group or an optionally protected guanine moiety,
R1 is a hydroxy protecting group or a -C(=O)C₁-C₂₂ alkyl ester group;
R2 and R3 are independently lower alkyl or benzyl, or R2 and R3 taken together are -CH₂CH₂- or-CH2CH2CH2- or -CH2CH2CH2CH2-;
to the corresponding aldehyde of the formula III: 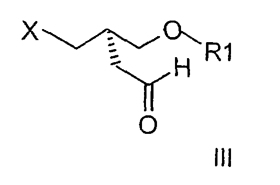 and the reduction of the aldehyde to the corresponding alcohol of the formula IV: 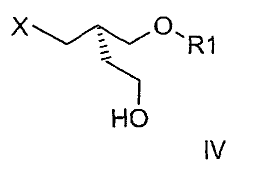 by the addition of a borohydride or borane aldehyde reducing agent,
**characterised in that** the aldehyde reducing agent is introduced under acid conditions.

The resultant alcohol IV is then esterified with an activated N-protected alpha amino acid derivative as described in the above prior art patent applications to form valomaciclovir stearate XX. If necessary the alcohol IV or its N-protected amino acyl derivative can be reacted with the guanine base as also described in the prior art and/or the alcohol protecting group R1 can be deprotected and replaced with a fatty acid ester by conventional acylation.

By the use of the present invention, the alcohol IV can be obtained at a purity greater than 85%, preferably greater than 90%. The purity of this intermediate has proven to contribute significantly to the overall yield of valomaciclovir stearate.

Preferably the process of the invention is carried out without isolating the intermediate aldehyde of formula III. An optional preliminary step may comprise the esterification of the hydroxy group of the corresponding alcohol I 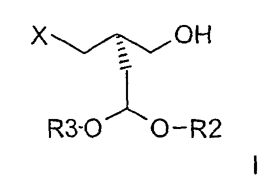 with an activated C₁-C₂₂COOH derivative, such as an activated stearic acid, by conventional acylation techniques, preferably without isolation of the resultant acetal II. Convenient activated derivatives for the esterification includes acid halides such as acid chlorides, and activated esters including, but not limited to, mixed anhydrides of steraric and pivalic and/or acetic acid derived anhydrides, anhydrides derived from alkoxycarbonyl halides such as isobutyloxycarbonylchloride and the like, N-hydroxysuccinimide derived esters, N-hydroxyphthalimide derived esters, N-hydroxybenzotriazole derived esters, N-hydroxy-5-norbornene-2,3-dicarboxamide derived esters, 2,4,5-trichlorophenyl derived esters, sulfonic acid derived anhydrides (for example, p-toluenesulonic acid derived anhydrides and the like) and the like. Alternatively the activated ester may comprise the acid in conjunction with a coupling agent such as DCC/DMAP or EDAC/DMAP.

Preferably, when R1 in the start material of formula IV is an alkanoic acid ester such as a stearic acid, the material is purified for excess stearic acid in order to minimize the production of undesired impurities, such as ( R)-9-[2-stearoyloxymethyl-4-(stearoyloxy)butyl]guanine. Purification may comprise one or more refluxing steps, for example between 10 minutes and 6 hours, such as 1 hour, in an organic solvent able to dissolve the alkanoic acid, such as acetone, typically followed by cooling to room temperature, filtration, washing with the same or similar solvent and drying. Conveniently the alkanoic acid content is reduced to < about 2%, preferably less than about 1%, such as <0.5%.

Following reaction of the borohydride or borane aldehyde reducing agent under acid conditions, it is convenient to neutralise the reaction mixture with bases such as 0.1 to 10.0 molar equivalents of sodium hydrogen carbonate, potassium carbonate, triethylamine, pyridine, NaOH KOH and the like, especially sodium bicarbonate.

Convenient strong acids for the hydrolysis of the acetal II include from about 0.1 to 10.0 molar equivalents of triflic, hydrochloric, formic or acetic/formic, sulphuric, sulphonic, p-toluene sulfonic or an ion exchanger sulphonic, especially hydrochloric. Solvents for the hydrolysis step include inert solvents such as THF/H₂O or methylene chloride/H₂O or ethylacetate/H₂O or ethanol/H₂O or methanol/H₂O or water, especially THF. Suitable reaction temperatures include from about -25°C to 100°C, such as room temperature

Convenient borohydrides for the reduction step include potassium borohydride and especially sodium borohydride. Convenient boranes include BH₃.py, 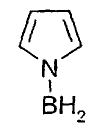 BH_{3.PY}, CF₃COOH, BH₃.NH₃, BH₃.NMe₃, BH₃.SMe₂,
and especially borane-tert-butylamine complex.

To minimize the formation of undesired regioisomers, such as (R )-9-[2-hydroxymethyl-4-stearoyloxybutyl]guanine by transesterification during the acid hydrolysis of the start material of formula II, when R1 is an alkanoic acid ester, it is convenient if the acid hydrolysis is made under mild conditions, such as at a low temperature, for a short time and/or with a low concentration of acid. It is furthermore convenient if the reaction mixture is neutralised as soon as all of the acetal of formula II has been hydrolysed and/or extra borohydride or borane aldehyde reducing agent is added to react with the last of the aldehyde present.

Convenient leaving groups for X in formulae I-IV include sulphonates such as methane sulphonate, triflate, p-toluenesulphonate, benzene sulphonate, especially TsO. Preferably, however, X in formulae I-IV is a guanine, a guanine derivative such as 6-deoxyguanine or 6-chloroguanine or iodoguanine, an N-protected and/or hydroxy-proteceted guanine such as 2-N-acetyl-guanine, 6-benzyloxyguanine or 2-N-acetyl-6-diphenyl-carbamoylguanine.

The term "lower alkyl" as used in conjunction with R2 and R3 refers to straight or branched chain alkyl radicals containing from 1 to 7 carbon atoms including, but not limited to, methyl, ethyl, n-propyl, iso-propyl, n-butyl, iso-butyl, sec-butyl, t-butyl, n-pentyl, 1-methylbutyl, 2,2-dimethylbutyl, 2-methylpentyl, 2,2-dimethylpropyl, n-hexyl and the like. Preferably R2 and R3 are the same species of lower alkyl, especially methyl and most preferably ethyl.

Preferred -C(=O)C₁-C₂₂ alkyl ester groups for R1 include acetyl, palmityl, cetyl, eicosanyl and especially stearyl. Alternative Hydroxy protecting groups for R1 are extensively discussed in Greene, "Protective Groups In Organic Synthesis," (John Wiley & Sons, New York (1981)). O-protecting groups comprise substituted methyl ethers, for example, methoxymethyl, benzyloxymethyl, 2-methoxyethoxymethyl, 2-(trimethylsilyl)ethoxymethyl, t-butyl, benzyl and triphenylmethyl; tetrahydropyranyl ethers; substituted ethyl ethers, for example, 2,2,2-trichloroethyl; silyl ethers, for example, trimethylsilyl, t-butyldimethylsilyl and t-butyldiphenylsilyl; and esters prepared by reacting the hydroxyl group with a carboxylic_acid, for example, acetate, propionate, benzoate and the like.

### Detailed Description

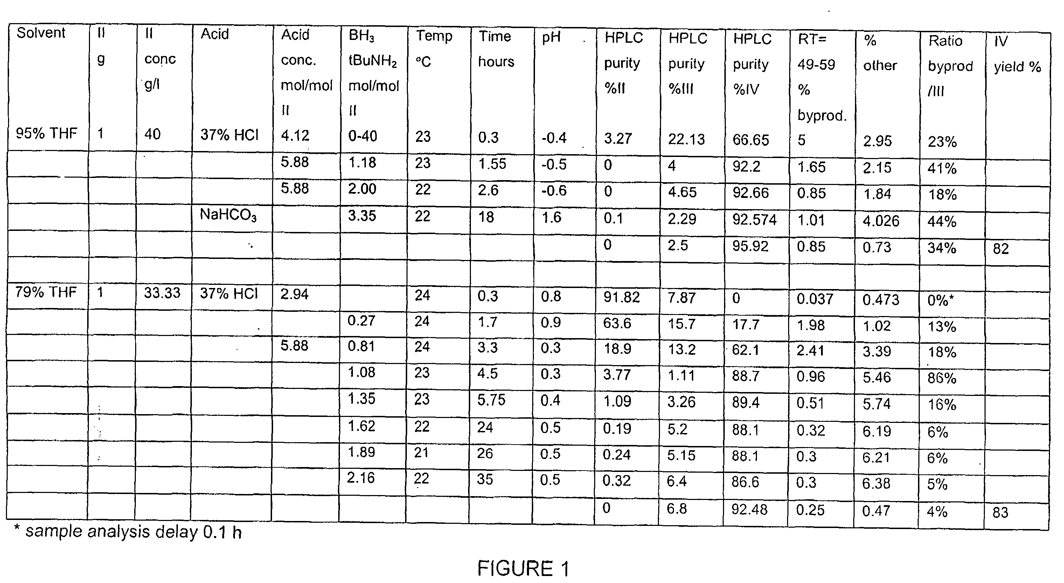

The invention will now be illustrated by way of example only with reference to Figure 1 which tabulates the results from Example 1

### Example 1

A flat bottomed reactor equipped with magnetic stirring, thermometer and a pH electrode was charged with:
1.0 g (1.7 mmol) (R)-9-[4,4-diethoxy-2-(stearyloxymethyl)butyl]guanine
95% tetrahydrofuran (25 ml)

The suspension was mixed for about 20 minutes to give a slightly turbid solution. To the solution was added portionwise over 4 hours:
37% hydrochloric acid (1.0 ml, 10 mmol)
Borane-tert-butyl-amine complex suspended in 3 ml 95% tetrahydrofuran
Borane-tert-butyl-amine complex (0.2 g, 2.3mmol).

The reaction mixture was adjusted to pH =1.6 by addition of:
0.4 g sodium hydrogen carbonate (4.8 mmol)
20 ml water

The next day the suspension was adjusted to pH=5 by addition of:
10ml water
3N sodium hydroxide (about 1 ml)

The stirred suspension was cooled and kept at 0°C for 1 hour. The suspension was filtered and the filter cake washed with acetone (2x2.5ml) and sucked dry. The wet filter cake was dried in a forced ventilation cupboard (50°C, 4.5 h) to give 0.72g (82% yield) of (R)-9-[4-hydroxy-2-(stearyloxymethyl)butyl]guanine with 95.9% purity. The above example and the corresponding trial with 79% THF is summarised in Table 1 in the accompanying Figure 1. The roman numeral II is the start material, III is the aldehyde and IV is the alcohol.

### Example 2Reduction of distearate

A 50 ml reaction flask with magnetic stirring was charged with:
Stearate alcohol (0.4 g, 0.77 mmol)
Tetrahydrofuran (27 ml)
3N HCl (3 ml, 9 mmol)

The stirred homogenous solution was kept at 41°C, while samples (25µl) were taken, diluted to 1.0 ml with 90% THF contaning 2.5% Et₃N and analysed by HPLC.

| T Time | Reaction Temp °C | Reaction time (h) | RT 16 min IV% | RT 21 min VII% | Total area AU*10⁸ |
|---|---|---|---|---|---|
| 12.58 | 20 | 0.00 | | | |
| 13.05 | 27 | 0.07 | 95.4 | 1.2 | 16.2 |
| 13.45 | 41 | 0.47 | | | |
| 14.10 | 41 | 1.12 | 89.1 | 8.6 | 13.9 |
| 15.15 | 41 | 2.17 | 79.6 | 16.85 | 10.7 |
| 17.00 | 41 | 4.02 | 69.4 | 25.88 | 7.7 |
| 12.25 | 41 | 23.27 | 31.9 | 28.4 | 0.48 |
| 9.10 | 41 | 68 | 0 | 0 | 0.18 |

It will be apparent that after 4 hours 25% of the regioisomer was formed by transesterification of the stearate alcohol, while 50% of the stearate alcohol and corresponding regioisomer were hydrolysed.. After about 23.5 h, about 3% of the stearate alcohol and regioisomer were present. The rest was apparently hydrolysed to stearic alcohol and guanine dialchohol. The conclusion is that the formation of the regioisomer is minimised if the hydrolysis of the acetal alkanoate is done under mild conditions, ie low temperature and/or short time and/or low acid concentration. Additionallly, it is helpful if the reaction mixture is neutralised without undue delay. Similarly, it assists if extra borane complex is added to react with any remaining aldehyde present.

### Example 3

### Preparation of (R)-9-(2-stearoyloxymethyl-4-hydroxybutyl)guanine

This example shows the preparation of the title compound from guanine alcohol without isolation of the intermediate guanine stearate and stearate aldehyde:

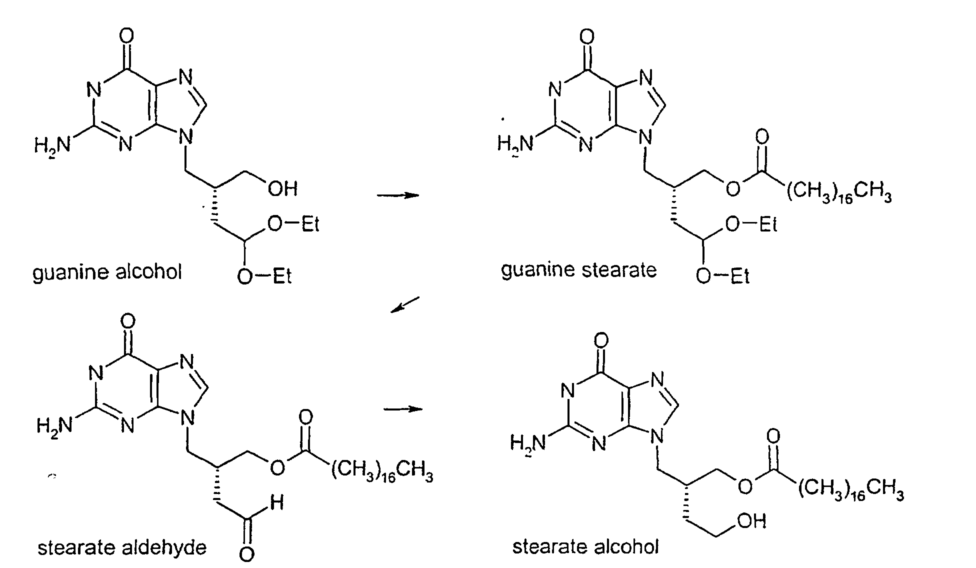

The following were charged in a reaction flask:
Stearic acid (26.0 g, 0.0913 mol), 99% pure
Tetrahydrofuran (500 g)
Triethyl amine (10.5 g, 0.104 mol)
Pivaloyl chloride (11.0 g, 0.913 mol), 99% pure
Guanine alcohol (27 g, 0.083 mol)
4-dimethylaminopyridine (2 g, 0.0163 mol) 99% pure

The stirred suspension was analysed after 2 days and found to contain 99.0% guanine stearate. Thereafter was added:
6N Hydrochloric acid (50 ml, 0.300 mol)
Borane-t-butyl amine complex (7.25 g, 0.083 mol) in portions of about 0.3 g over two hours, while the temperature was maintained at about 24°.

To this reaction mixture was added:
Water 800 ml
1.2N Ammonium hydroxide (195 ml, 0.234 mol) over 10 minutes. The pH was 5.2. The white suspension was colled and kept at 5°C for 2 hours and filtered on a G3 glass filter over 1.5 hours. The filter cake was washed with acetone (2x50 ml) and the wet filter cake (101 g) dried at 50°C overnight, yielding 43.5 g (97%) stearate alcohol at 75.7% purity with 23.3% guanine stearate and 0.5% stearate aldehyde.

To reduce the guanine stearate content, it was charged into a reaction flask:
43 g of the material from the preceding step
Tetrohydrofuran (600 g)
Water (50 g)
6N sulphuric acid (50 ml)
Borane t-butyl amine complex (4.5 g, 0.052 mol) in 0.3 g portions was added over 7 hours while maintaining the temperature at about 24°C. The reaction was monitored for completion by HPLC, the last point being 0.18% guanine stearate. The reaction mixture was worked up as previously described to yield 39.54 g stearate alcohol, purity 94.4%, 2.6% stearate aldehyde and 0,17% guanine stearate.

The product was cleaned twice by reflux in acetone. In a 500 ml falsk was placed:
Acetone (400 ml)
Product (31.77 g)

The stirred suspension was heated and kept at reflux for 1 hour, cooled to 27°C, filtered and washed with acetone (3x25 ml). The procedure was repeated. The resulting wet filter cake (33.9g) was dried at 50°C for 16 hours to give 27.9 g (82% relative to guanine alcohol) at 95.0% stearate alcohol, 0.1% guanine stearate, 2.7% stearate alcohol regioisomer, 1.4% of a product with RT=50-58 minutes and 2% steraric acid.

Additional runs were performed using dried gaunaine alcohol, HCI and borane t-butylamine complex. The wet filtercake of stearate alcohol was refluxed twice with acetone to remove stearic acid:

| | | |
|---|---|---|
| Material & step Stearic acid | 30.2 g | 75.5 g |
| Tetrahydrofuran | 535 g | 1338 g |
| Triethylamine | 14.8 g | 37 g |
| Pivaloyl chloride | 12.4 g | 31 g |
| Guanine alcohol | 27 g | 67.5 g |
| Dimethylaminopyridine | 2 g | 5 g |
| Reaction time | 18 hours | 24 hours |
| 3N HCI | 110 ml | 275 ml |
| Borane t-butyl amine complex | 9.8 g | 27 g |
| Temp. range during hydrolysis & reduction | 29-33°C | 25-17°C |
| Sodium hydrogen carbonate | 15 g | 26 g |
| Water | 1000 ml | 2500 ml* |
| Borane-t-butyl amine complex | 1 g | 4.5 g |
| Reflux with acetone | 1000 ml | 2500 ml |
| Content of stearic acid in acetone wash | 4.9 g | 9.9 g |
| Reflux with acetone | 1000 ml | 2500 ml |
| Content of stearic acid in acetone wash | 2.5 g | 2.0 g |
| Yield of dry stearate alcohol | 37.85,88% | 95.45 g, 89% |
| Purity | 96.8% | 97.5% |
| Content of stearic acid | 0% | 0% |

| | | |
|---|---|---|
| * HPLC indicated unreacted guanine stearate, so subjected to additional HCl/borane-t-butylamine complex | | |

### Example 4

### Avoiding regioisomerism during hydrolysis

In a 50 ml reaction flaks with magnetic stirring was charged:
Stearate alcohol (0.4 g, 0.77 mmol)
Tetrahydrofuran (27 ml)
3N HCI (3ml, 9 mmol)

The stirred homogenous solution was kept at 41°C. 25 µl sampes were taken, diluted to 1.0 ml with 90% THF containing 2.5% ET₃N and analysed by HPLC:

| Reaction temp °C | Reaction time hours | RT 16 min stearate alcohol % | RT 21 min Regioisomer % | Total area AU*10⁸ |
|---|---|---|---|---|
| 20 | 0:00 | | | |
| 27 | 0:07 | 95.4 | 1.2 | 16.2 |
| 41 | 0:47 | | | |
| 41 | 1.12 | 89.1 | 8.6 | 13.9 |
| 41 | 2:17 | 79.6 | 16.85 | 10.7 |
| 41 | 4:02 | 69.4 | 25.88 | 7.7 |
| 41 | 23.27 | 31.9 | 28.4 | 0.48 |
| 41 | 68 | 0 | 0 | 0.18 |

After 4 hours, 25% of regiosiomer (R )-9-(2-hydroxymethyl-4-stearoyloxy)butylguanine had formed by transesterification of the intended stearate alcohol, while about 50% of the stearate alcohol and regioisomer had hydrolysed. After 23.5 hours, about 3% of the intended stearate alcohol and regioisomer were present. The rest was apparently hydrolysed to stearic acid and guanine dialcohol. It is thus apparent that the formation of the regioisomer is minimized if the acid hydrolysis of the guanine stearate is performed under mild conditions, that is at low temperature and/or short time and/or low acid concentration. Furthermore it may be advantageous that the reaction mixture is neutralised as soon as the guanine stearate is all hydrolysed and/or extra borane reagent added with any remaining stearate alcohol present.

Plots (not depicted) of analystic results as a function of reaction time were drawn from further dual experiments starting with guanine stearate (1 g, 1.7 mmol) dissolved in 90% THF (25 ml) and acid (10 mmol). BH₃, t-BuNH₂ (200 mg, 2.3 mmol) was added at constant rate, while the temperature was kept at 30°C. Samples (25 µl) were taken for HPLC analysis of guanine stearate, stearate aldehyde, stearate alcohol and guanine alcohol during the 4-5 hour reaction time. Both plots were similar with an intitial fast hydrolysis of guanine stearate, a high concentration of stearate aldehyde, and a linear increase in stearate alcohol, governed by the addition rate of BH₃, t-BuNH₂.

### Example 5

### Preparation of pure guanine stearate

To prepare stearic acid free guanine stearate, an experiment was run with only 90 mol% staeric acid and pivaloyl chloride. The starting materials were stearic acid (21.3 g, 75 mmol), pivaloyl chloride (9.0 g, 75 mmol) and guanine alcohol (27 g, 83 mmol). The yield was 33.5 g (75% relative to stearic acid) of guanine stearate, purity 99.6% (HPLC). A stearic acid determination gave < 1%, as expected, as the guanine stearate is precipitated by addition of acetone (1000 ml) which will dissolve stearic acid.

The filtrate (1350 ml) contained stearic acid 4.8 g, 17 mmol) corresponding to 23% of the charged amount. This could be explained by water present in the reaction mixture. The solvent tetrahydrofuran was almost anhydrous (6 mg H₂O/l) but the guanine alcohol contained 28 mg H₂/l, which corresponds to ½ mol crystal water. This means that the 27 g of guanine alcohol contained 42 mmol H₂, which could react with the mixed anhydride of astearic acid and pivalic acid. Accordingly, it is advantageous if the guanine alcohol start material is dried by to reaction, for instance at 50°C for 2 hours to reduce the water content to less than 20 mg H₂O/g, preferably less than 10 mg/g, most preferably < 5 mg/g

## Claims

1. A method comprising the acid hydrolysis of an intermediate of the formula II: 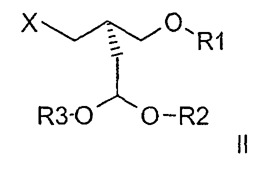 where X is a leaving group or an optionally protected guanine moiety,
R1 is a hydroxy protecting group or a -C(=O)C₁-C₂₂ alkyl ester group;
R2 and R3 are independently lower alkyl or benzyl, or R2 and R3 taken together are -CH₂CH₂- or -CH2CH2CH2-or -CH2CH2CH2CH2-;
to the corresponding aldehyde of the formula III: 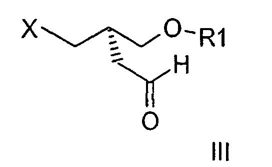 and the reduction of the aldehyde to the corresponding alcohol of the formula IV: 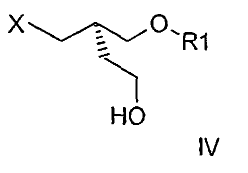 by the addition of a borohydride or borane aldehyde reducing agent,
**characterised in that** the borohydride or borane aldehyde reducing agent is introduced under acid conditions.

2. The method of claim 1, wherein the aldehyde reducing agent is introduced at a pH below 1.6.

3. The method of claim 1, wherein the aldehyde reducing agent is borane-tert-butylamine complex.

4. The method of claim 1, wherein the reaction is carried out in a solvent comprising tetrahydrofuran.

5. The method of claim 1, further comprising a pre-step of acylating a compound of the formula I 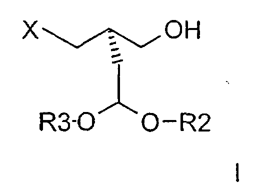 with an activated C₁-C₂₂COOH derivative to produce said compound of formula II.

6. The method of claim 5, wherein the activated derivative is an activated stearic acid.

7. The method of claim 5, wherein the compound of formula II is not isolated.

8. The method of claim 7, wherein the aldehyde of formula III is not isolated.

9. The method of claim 1, wherein the aldehyde of formula III is not isolated.

10. The method of claim 1 wherein X is a guanine moiety or an N-protected and/or hydroxy-protected guanine moiety.

11. The method of claim 1 wherein R2 and R3 are each methyl or ethyl.

12. The method of claim 1 wherein R1 is stearyl ester.

13. The method of claim 1 wherein R1 is acetyl ester.

14. The method of claim 1, wherein the water content of the start material of formula II is less than 20 mg H₂O/g start material, preferably less than 10 mg/g, most preferably < 5 mg/g.

15. The method of claim 5, further comprising the step of removing unreacted acyl derivatives.

16. The method of claim 15, wherein the content of unreacted acyl derivatives is controlled to less than 2% dry weight, relative to the dry weight of the reaction products.

17. The method of claim 15, wherein the acyl removal comprises washing with an organic solvent that dissolves the unreacted acyl derivatives, but precipitates the acyl ester of formula II.

18. The method of claim 17, wherein the solvent is acetone.

## Patentansprüche

1. Verfahren, umfassend die Säurehydrolyse eines Intermediats der Formel II: 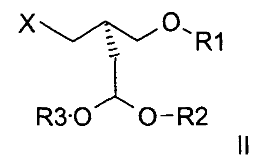 in der X eine Abgangsgruppe oder ein gegebenenfalls geschützter Guanin-Rest,
R1 eine Hydroxy-Schutzgruppe oder eine -C(=O)C₁-C₂₂ Alkylestergruppe ist;
R2 und R3 unabhängig voneinander niedrigere Alkyl oder Benzyl, oder R2 und R3 zusammen -CH₂CH₂- oder-CH2CH2CH2- oder -CH2CH2CH2CH2- sind;
zu dem entsprechenden Aldehyd der Formel III: 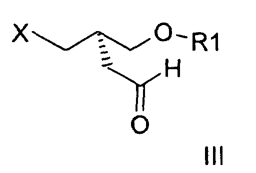 und die Reduktion des Aldehyds zum entsprechenden Alkohol der Formel IV: 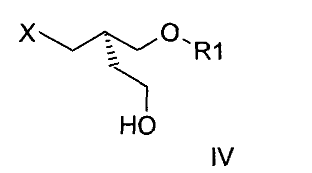 durch die Zugabe eines Borhydrid oder Boranaldehyd reduzierenden Mittels,
**dadurch charakterisiert, dass** das Borhydrid- oder Boranaldehyd reduzierende Mittel unter Säurebedingungen eingeführt wird.

2. Verfahren gemäß Anspruch 1, wobei das den Aldehyd reduzierende Mittel bei einem pH unter 1,6 eingeführt wird.

3. Verfahren gemäß Anspruch 1, wobei das den Aldehyd reduzierende Mittel ein Boran*tert*-butylamin-Komplex ist.

4. Verfahren gemäß Anspruch 1, wobei die Reaktion in einem Lösungsmittel ausgeführt wird, das Tetrahydrofuran enthält.

5. Verfahren gemäß Anspruch 1, weiter umfassend einen vorgeschalteten Schritt, eine Verbindung der Formel I 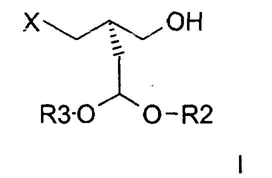 mit einem aktivierten C₁-C₂₂COOH-Derivat zu acylieren, um so die Verbindung der Formel II herzustellen.

6. Verfahren gemäß Anspruch 5, wobei das aktivierte Derivat eine aktivierte Stearinsäure ist.

7. Verfahren gemäß Anspruch 5, wobei die Verbindung der Formel II nicht isoliert wird.

8. Verfahren gemäß Anspruch 7, wobei der Aldehyd der Formel III nicht isoliert wird.

9. Verfahren gemäß Anspruch 1, wobei der Aldehyd der Formel III nicht isoliert wird.

10. Verfahren gemäß Anspruch 1, wobei X ein Guanin-Rest oder ein N-geschützter und/oder Hydroxy-geschützter Guanin-Rest ist.

11. Verfahren gemäß Anspruch 1, wobei R2 und R3 jeweils Methyl oder Ethyl sind.

12. Verfahren gemäß Anspruch 1, wobei R1 ein Stearylester ist.

13. Verfahren gemäß Anspruch 1, wobei R1 ein Acetylester ist.

14. Verfahren gemäß Anspruch 1, wobei der Wassergehalt des Ausgangsmaterials der Formel II weniger als 20 mg H₂O/g Ausgangsmaterial, vorzugsweise weniger als 10 mg/g, am bevorzugtesten < 5 mg/g beträgt.

15. Verfahren gemäß Anspruch 5, weiter umfassend den Schritt, nicht umgesetzte Acyl-Derivate zu entfernen.

16. Verfahren gemäß Anspruch 15, wobei der Gehalt an nicht umgesetzten Acyl-Derivaten auf weniger als 2% Trockengewicht, im Verhältnis zum Trockengewicht der Reaktionsprodukte, eingestellt wird.

17. Verfahren gemäß Anspruch 15, wobei die Acyl-Entfernung das Waschen mit einem organischen Lösungsmittel umfasst, das die nicht umgesetzten Acyl-Derivate auflöst, aber den Acylester der Formel II präzipitiert.

18. Verfahren gemäß Anspruch 17, wobei das Lösungsmittel Aceton ist.

## Revendications

1. Procédé comprenant l'hydrolyse acide d'un intermédiaire de formule II: 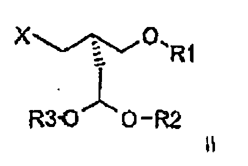 dans lequel X est un groupe partant ou une partie guanine éventuellement protégée,
R1 est un groupe protecteur d'hydroxy ou un groupe ester de -C(=O)C₁-C₂₂ alkyle ;
R2 et R3 sont, indépendamment, un alkyle inférieur ou un benzyle, ou R2 et R3, pris ensemble, sont -CH₂CH₂- ou -CH₂CH₂CH₂- ou -CH₂CH₂CH₂CH₂- ;
en l'aldéhyde correspondant de formule III: 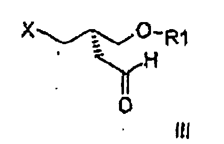 et la réduction de l'aldéhyde en l'alcool correspondant de formule IV: 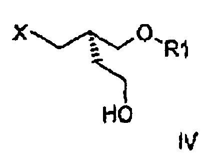 par l'ajout d'un agent réducteur d'aldéhyde borohydrure ou borane,
**caractérisé en ce que** l'agent réducteur d'aldéhyde borohydrure ou borane est introduit dans des conditions acides.

2. Procédé selon la revendication 1, dans lequel l'agent réducteur d'aldéhyde est introduit à un pH inférieur à 1,6.

3. Procédé selon la revendication 1, dans lequel l'agent réducteur d'aldéhyde est un complexe borane-tert-butylamine.

4. Procédé selon la revendication 1, dans lequel la réaction est réalisée dans un solvant comprenant du tétrahydrofurane.

5. Procédé selon la revendication 1, comprenant en outre une étape préalable d'acétylation d'un composé de formule I 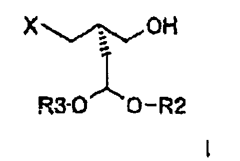 avec un dérivé C₁-C₂₂COOH activé pour produire ledit composé de formule II.

6. Procédé selon la revendication 5, dans lequel ledit dérivé activé est un acide stéarique activé.

7. Procédé selon la revendication 5, dans lequel le composé de formule II n'est pas isolé.

8. Procédé selon la revendication 7, dans lequel l'aldéhyde de formule III n'est pas isolée.

9. Procédé selon la revendication 1, dans lequel l'aldéhyde de formule III n'est pas isolée.

10. Procédé selon la revendication 1, dans lequel X est une partie guanine ou une partie guanine protégée par N et/ou par un hydroxy.

11. Procédé selon la revendication 1, dans lequel R2 et R3 sont chacun méthyle ou éthyle.

12. Procédé selon la revendication 1, dans lequel R1 est un ester de stéaryle.

13. Procédé selon la revendication 1, dans lequel R1 est un ester d'acétyle.

14. Procédé selon la revendication 1, dans lequel la teneur en eau du matériau de départ de formule II est inférieure à 20 mg de H₂O/g de matériau de départ, de préférence inférieure à 10 mg/g, tout préférentiellement < 5 mg/g.

15. Procédé selon la revendication 5, comprenant en outre l'étape consistant à éliminer les dérivés acyle n'ayant pas réagi.

16. Procédé selon la revendication 15, dans lequel la teneur en dérivés acyle n'ayant pas réagi est maintenue en dessous de 2% du poids sec, par rapport au poids sec des produits de la réaction.

17. Procédé selon la revendication 15, dans lequel l'élimination des acyle comprend le lavage avec un solvant organique qui dissout les dérivés acyle n'ayant pas réagi, mais qui précipite l'ester d'acyle de formule II.

18. Procédé selon la revendication 17, dans lequel le solvant est l'acétone.
